# EUROPEAN PATENT SPECIFICATION

(11) **EP 2 599 563 B1**
(45) Date of publication and mention of the grant of the patent: **07.10.2015**
(21) Application number: 12193567.0
(22) Date of filing: 21.11.2012
(51) Int. Cl.: B08B 3/04, A61L 2/00, B08B 9/00, A61L 9/14

(54) **Apparatus for hygienising thermal conditioning stations**
Vorrichtung zur Desinfektion von Wärmekonditionierungsstationen
Appareil de conditionnement thermique d'hygiénisation de stations

(30) Priority: 29.11.2011 IT MO20110307
(43) Date of publication of application: 05.06.2013
(73) Proprietor: Rational Production S.r.l., 24061 Albano Sant Alessandro (IT)
(72) Inventor: Cattaneo, Ivan, 24060 Carobbio degli Angeli (Bergamo) (IT); Cattaneo, Diego, 24020 Ranica (Bergamo) (IT)
(74) Representative: Crugnola, Pietro

(56) References cited:
- WO-A1-93/18249
- WO-A1-94/15501
- DE-A1- 4 234 062
- DE-A1- 19 952 330
- DE-U1- 20 304 952
- FR-A1- 2 681 853
- US-B2- 7 152 664

## Description

The invention relates to a hygienising apparatus, in particular a movable hygienising apparatus arranged for performing hygienising cycles comprising washing, sanitising and/or drying cycles in a thermal conditioning station.

The thermal conditioning station, in use, is coupled with a carriage arranged for supporting trays, or other types of container intended for containing food, for example for patients in a hospital facility, and for transporting and distributing said trays or other types of container.

The carriage that supports trays or other types of container comprises a body mounted on wheels, to make the body movable, inside which a loading chamber is defined comprising at least one chamber containing the trays or other types of container to be transported and distributed.

The thermal conditioning station has the object of maintaining or bringing at least one chamber of the body of the carriage that supports trays or other types of container to a preset temperature, after the thermal conditioning station and the carriage have been coupled in such a manner that the chamber of the carriage and an internal space of the thermal conditioning station are flowingly connected.

The loading chamber can also comprise a plurality of chambers that can be maintained or taken to temperatures that are different from one another, the chambers being separated from one another by a vertical separating wall. In this case the thermal conditioning station maintains or takes each chamber to the corresponding preset temperature. For example, the vertical separating wall can divide the loading chamber of the carriage into two chambers, one intended for containing the food to be served hot and the other intended for containing the food to be served cold. On the vertical separating wall horizontal shelves are obtained for housing the trays or the other types of food container. The vertical separating wall thus enables the trays or the other types of container to be divided into two parts, in the two chambers of the loading chamber. The vertical separating wall is provided with sealing means to enable the two chambers to be maintained at or taken to two different temperatures, when the carriage is coupled with the thermal conditioning station.

The sealing means can comprise a magnetic coupling device provided with a plurality of seals arranged on a contact perimeter between the thermal conditioning station and the carriage, in such a manner as to ensure an air seal between the two elements.

The thermal conditioning station is fixed, for example secured to a wall of a service room of the hospital facility. The thermal conditioning station comprises thermal conditioning means able to generate flows of hot and/or cold air to be conveyed inside the carriage, once coupled.

In order to carry out coupling with the carriage, a hatch is opened that is arranged on a coupling side of the thermal conditioning station and the carriage has to be moved towards the latter, in particular towards a coupling side thereof. With reference to cases in which the carriage has a loading chamber divided into two chambers, once the carriage and the thermal conditioning station have been coupled, the thermal conditioning station first performs a maintenance step during which it generates flow of cold air for each of the chambers defined by the separating wall and maintains at about 3°C the food positioned on the trays or in other types of container. Subsequently, the thermal conditioning station performs a regenerating step during which it generates a flow of cold air conveyed to the chamber where the food to be consumed cold has been introduced, and a flow of hot air conveyed to the other chamber where the food to be consumed hot has been introduced.

The sealing means enables an escape of air from the semi-chambers to the outside environment to be avoided, and mixing between the flow of hot air and the flow of cold air to be avoided.

The flow of cold air and the flow of hot air are produced by the thermal conditioning station using the thermal conditioning means, which can comprise fans, electrical resistances, finned exchangers, and, lastly, conveying and protection panels.

The thermal conditioning means is located in a part of the thermal conditioning station intended for being placed in communication with the carriage, once coupled; this part has a width and a height that are substantially similar to the width and to the height of the loading chamber of the carriages to be coupled.

The internal space of the thermal conditioning station is divided into two portions separated vertically by a dividing baffle, provided with a vertical sealing gasket, intended, in use, to bring into contact an end portion of the vertical separating wall of the two chambers of the carriage, when the latter and the thermal conditioning station are coupled. In this manner, a thermal separation is achieved between the two portions of the internal space of the thermal conditioning station, intended respectively for the one generating a flow of cold air and for the other first maintaining, in the cold maintenance step, a cold flow, subsequently, in the regenerating step, a flow of hot air.

Periodically, the thermal conditioning station has to be hygienised, i.e. it has to undergo washing, sanitising and/or drying operations performed by a hygienising apparatus, in order to be able return to the optimum hygienic operating conditions.

As disclosed above, the thermal conditioning station is fixed stably to a wall of rooms situated inside the in-patient wards, cannot be washed with a hygienising apparatus that generates jets of water, not even in parts where this would be technically possible.

Such hygienising operations have to be performed by an operator using a hygienising apparatus of known type on suitable rooms far from the in-patient wards, where it is possible to transfer periodically the thermal conditioning station to perform the hygienising operations.

This hygienising apparatus comprises, for example, a portable generator device arranged for generating jets of steam that are suitable for cleaning the internal space of the thermal conditioning station and is provided with a pipe for guiding the jets inside the internal space of the thermal conditioning station.

In this case, nevertheless, no administration of a hospital facility would assume the responsibility of delegating to an operator entrusted with such operations the care not to damage parts that are unprotected from water. Further, conveying the thermal conditioning station to a suitable room would mean need to have another substitute thermal conditioning station that replaces the station subjected to hygienising operations. Still further, this would mean having to set up equipped rooms to perform such hygienising operations. Lastly, the hygienising operations take a significant time owing to the fact that the operator entrusted with said operations has to transport the thermal conditioning station to the equipped room, which may be a considerable distance from the place in which the thermal conditioning station operates.

Alternatively, the aforesaid hygienising operations are conducted in loco by the operator, who used the hygienising apparatus of known type.

A drawback of such hygienising apparatuses is that the hygienising operations that they perform are ineffective, because of the impossibility of reaching with the steam jets zones located on the opposite side of the conveying panels of the thermal conditioning means with respect to where the steam jets are projected and owing to the impossibility of saturating with steam the internal space because it is open to the external environment.

Further, the efficacy of the steam as a sanitising agent is a function of the jet temperature, which is appropriate only for the surfaces affected directly; in fact, the steam, that hits surfaces of evaporators sheltering behind the conveying panels and which has cooled by hitting other surfaces that it meets along the path to reach all the zones of the internal space and which are colder than the temperature of the steam, is not as effective as the steam that has just be generated. An object of the invention is to overcome the drawbacks of the hygienising apparatuses of thermal conditioning stations of known type.

Another object of the invention is to obtain a hygienising apparatus of thermal conditioning stations that enables effective hygienising operations to be performed on said thermal conditioning stations.

A further object of the invention is to obtain a hygienising apparatus of thermal conditioning stations that is portable and is able to operate on said thermal conditioning stations existing in the place in which they exist.

A still further object of the invention is to obtain a hygienising apparatus of thermal conditioning stations shortens hygienising time.

According to the invention, a hygienising apparatus of thermal conditioning stations as defined in claim 1 is provided.

Owing to the invention, it is possible to obtain a hygienising apparatus of thermal conditioning stations that performs effective and fast hygienising operations and enables said operations to be performed without moving the thermal conditioning stations from the place in which they are located.

The invention can be better understood and implemented with reference to the attached drawings that illustrate some embodiments thereof by way of non-limiting example, in which:
Figure 1 is a perspective view of a hygienising apparatus of thermal conditioning stations according to the invention coupled with a thermal conditioning station;
Figure 2 is a perspective view of a thermal conditioning station;
Figure 3 is a view like the one in Figure 1 in which parts of the hygienising apparatus of thermal conditioning stations have been removed to enable other parts of the hygienising apparatus to be viewed;
Figure 4 is a view like the one in Figure 3 taken from another angle;
Figure 5 is an enlarged detail in Figure 1;
Figure 6 is an enlarged detail in Figure 5;
Figure 7 is a top view of the hygienising apparatus of thermal conditioning stations according to the invention;
Figure 8 is a section taken along the plane VIII-VIII in Figure 7;
Figure 9 is a section taken along the plane IX-IX in Figure 7;

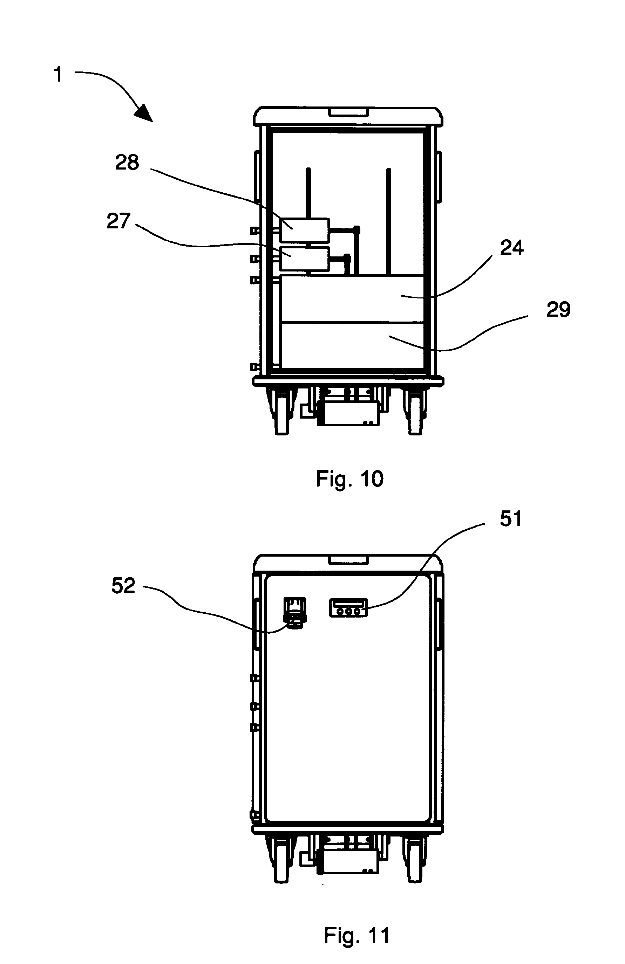

Figure 10 is a section taken along the plane X-X in Figure 7;
Figure 11 is a frontal view of the hygienising apparatus of thermal conditioning stations according to the invention;
Figure 12 is a perspective view of hygienising means with which the hygienising apparatus according to the invention is provided;
Figure 13 is an enlarged detail in Figure 12;
Figure 14 is a view highlighting spraying means of the hygienising means with which the hygienising apparatus according to the invention is provided;
Figure 15 is a view of a refrigerating unit and of an embodiment of the sanitising means of the hygienising means with which the hygienising apparatus according to the invention is provided;
Figure 16 is an enlarged detail of the refrigerating unit in Figure 15;
Figure 17 is another enlarged detail of the sanitising means in Figure 15;
Figure 18 is a view of another embodiment of the sanitising means with which the hygienising apparatus is provided;
Figure 19 is an enlarged detail in Figure 18;
Figure 20 is a view of a further embodiment of the sanitising means with which the hygienising apparatus is provided;
Figure 21 is an enlarged detail in Figure 20.

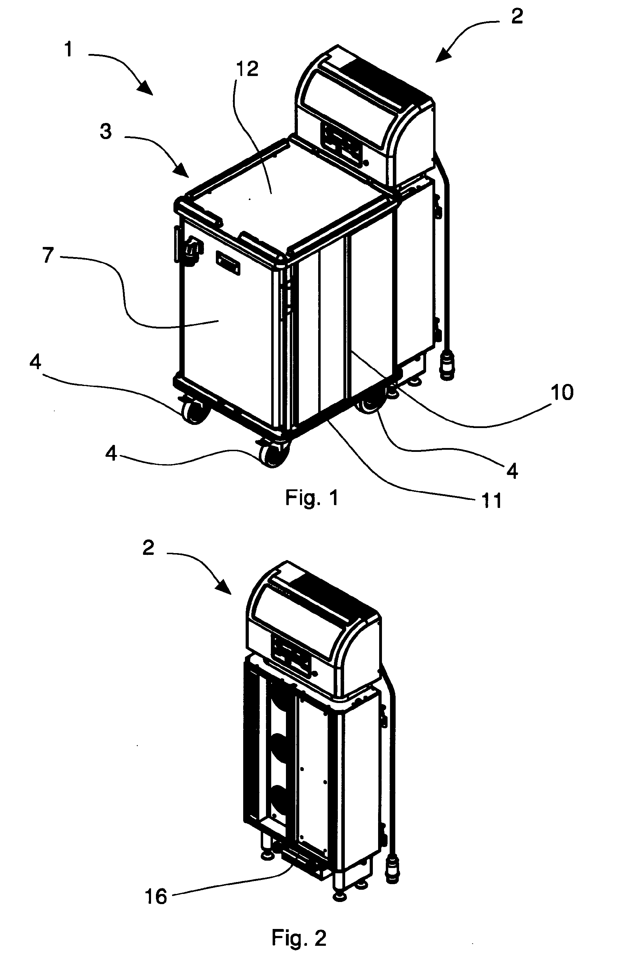

With reference to Figures 1 to 4 a hygienising apparatus 1 of thermal conditioning stations coupled with a thermal conditioning station 2 is shown.

The thermal conditioning station 2 is further arranged for being coupled to a carriage, which is not illustrated, arranged for supporting trays or other types of container intended for containing food, for example for in-patients of a hospital facility, and for conveying and distributing said trays or other types of container.

The thermal conditioning station 2 has the object of maintaining or taking to a preset temperature at least one internal chamber with which the carriage is provided that supports trays or other types of container after the thermal conditioning station 2 and the carriage have been coupled in such a manner that the internal chamber of the carriage and an internal space 20 of the thermal conditioning station 2 are flowingly connected.

The thermal conditioning station 2 comprises thermal conditioning means that is able to generate flows of hot and/or cold air. Such flows of hot and/or cold air can be conveyed inside the carriage, or, as we will see better below, inside the hygienising apparatus 1, once they have been coupled with the thermal conditioning station.

The flow of cold air and the flow of hot air are produced by the thermal conditioning station 2, respectively in a cold side and a hot side of the internal space 20, by using the thermal conditioning means, which can comprise fans, electrical resistances, finned heat exchangers, and, lastly, conveying and protection panels.

The thermal conditioning means is located in a part of the thermal conditioning station 2 intended for being placed in communication with the carriage or with the hygienising apparatus 1, once coupled.

The internal space 20 of the thermal conditioning station 2 is divided into two portions separated vertically by a dividing baffle, provided with a vertical sealing gasket, intended, in use, for bringing into contact an end portion of the vertical separating wall of the carriage, when the latter and the thermal conditioning station are coupled. In this manner, a thermal separation is achieved between the two portions of the internal space of the thermal conditioning station 2, the one intended respectively to generate a flow of cold air, and the other first a cold air flow, and subsequently, a hot air flow.

The thermal conditioning station 2 is normally fixed stably to a wall of the room in which it is located, for example a room of a hospital facility.

The thermal conditioning station 2 is provided with at least one door arranged on the coupling side, that has to be taken to an open position when the thermal conditioning station 2 has to be coupled with a carriage or with a hygienising apparatus 1.

The hygienising apparatus 1 is arranged for periodically running a hygienising cycle, which comprises, for example, a washing cycle, a sanitising cycle and/or a drying cycle, on the thermal conditioning station 2, when the latter is coupled with the hygienising apparatus 1.

The hygienising cycle has the function of returning the thermal conditioning station 2 to optimum hygienic operating conditions.

The hygienising apparatus 1 comprises a box body 3, for example of parallelpipedon shape, mounted on corresponding movement means comprising, for example, a plurality of wheels 4 that enable the hygienising apparatus 1 to be moved.

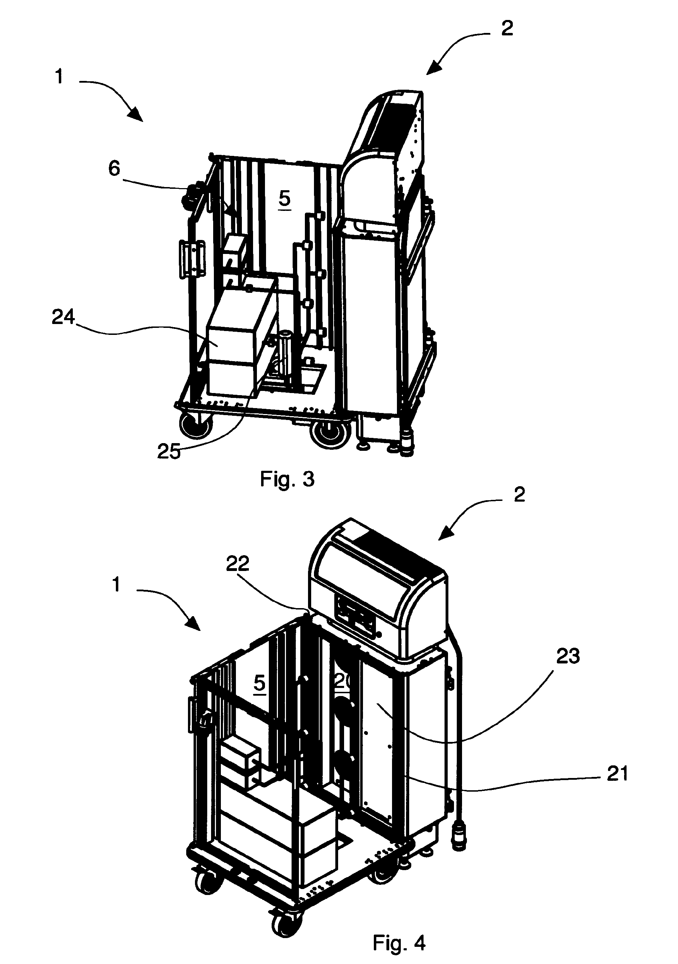

The box body 3 defines a chamber 5, shown, for example, in Figure 3, intended for housing hygienising means 6 of the hygienising apparatus 1. This hygienising means 6 is suitable for performing the aforesaid hygienising cycle.

The box body 3 comprises a front wall 7, a rear wall 8, opposite said front wall 7, and a pair of reciprocally opposite side walls 10.

The box body 3 further comprises a base 11, to which the wheels 4 and a cover 12 are connected.

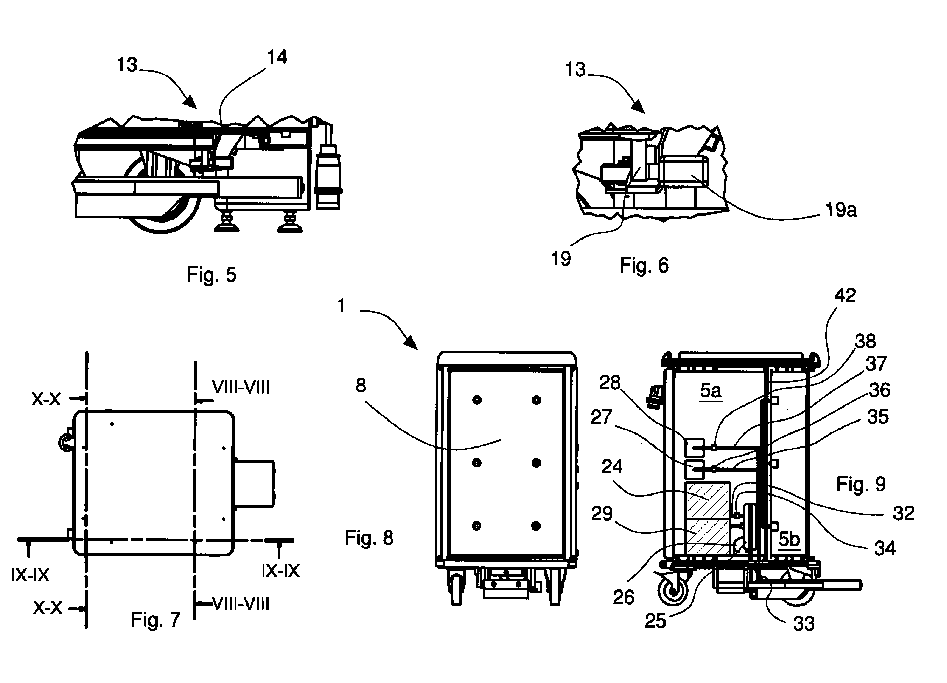

The hygienising apparatus 1 further comprises coupling means 13, shown in particular in Figures 5 and 6, arranged for enabling the hygienising apparatus 1 to couple with the thermal conditioning station 2.

The coupling means 13 can be connected to the base 11 and, in particular, can extend below the base 11. Further, the coupling means 13 is arranged near a lower rear edge 14 of the base 11, i.e. the edge that faces the thermal conditioning station 2 when the hygienising apparatus 1 couples with the thermal conditioning station 2.

The coupling means 13 can comprise a block 15, made of a ferromagnetic material, arranged for interacting with an electromagnet 16, shown in Figure 2, located on the thermal conditioning station 2.

The block 15 and the electromagnet 16 are arranged in such a manner that, when the rear wall 8 of the hygienising apparatus 1 is brought near the thermal conditioning station 2, they face one another. In this manner, the action of the electromagnet 16 on the ferromagnetic material of the block 15 enables the hygienising apparatus 1 and the thermal conditioning station 2 to be kept joined together until the supply is disconnected from the electromagnet 16.

In an alternative embodiment, that is not shown, the coupling means 13 of the hygienising apparatus 1 can comprise the electromagnet 16 whilst the thermal conditioning station 2 can be provided with the block 15 of ferromagnetic material. When the coupling means 13 is driven, the chamber 5 and the internal space 20 of the thermal conditioning station 2 become a single flowingly connected environment.

The hygienising apparatus 1 can be provided with aligning means that is not shown to facilitate coupling with the thermal conditioning station 2, which is provided with further aligning means, which is not shown.

Such aligning means and further aligning means can comprise bar adhesives, arranged, respectively, on the cover 12 and on a top wall of the thermal conditioning station 2, said aligning means and said further aligning means being positioned on a same longitudinal axis to visually help the operator to move the hygienising apparatus 1 near the thermal conditioning station 2 in an aligned position and thus enable effective coupling.

In order to avoid a leak of fluid from the single environment to the external environment, the hygienising apparatus 1 is provided with sealing means 21. The sealing means 21 comprises a seal or a plurality of seals arranged on a perimeter of the rear wall 8 intended for contacting the thermal conditioning station 2.

The sealing means 21, in use, interacts with corresponding further sealing means 22 arranged on a perimeter of a front face 23 of the thermal conditioning station 2 in such a manner as to ensure an air and water seal between the two elements.

The hygienising apparatus 1 comprises recognition means arranged such that the thermal conditioning station 2 can recognise the hygienising apparatus 1 as such and not confuse it with the carriage supporting trays or other types of container intended for containing food.

The recognition means can comprise an abutting element 19 arranged on the hygienising apparatus 1, suitable for interacting with a microswitch 19a arranged on the thermal conditioning station 2 in such a manner as to be activated only by the hygienising apparatus 1 and not by the carriage. Th abutting element 19 and the microswitch 19a are shown, for example, in Figure 6.

Alternatively, the microswitch 19a can be arranged on the hygienising apparatus and the abutting element 19 on the thermal conditioning station 2.

In an alternative embodiment, which is not illustrated, the recognition means can comprise an RFID device that is read by a reading unit, which is not illustrated, of the thermal conditioning station 2.

Alternatively, the RFID device can be located on the thermal conditioning station 2.

Also in an alternative embodiment, which is not illustrated, it is a management and control unit of the hygienising apparatus 1, for example of wireless type, that acts as a recognition means, inasmuch as it is able to dialogue by exchanging signals with a further management and control unit of the thermal conditioning station 2.

The hygienising means 6 comprises a first tank 24 arranged for containing water to be used in the washing cycle.

The first tank 24 is provided with a connecting element, such as a quick connector, for connection to the mains water supply of the room in which the hygienising apparatus 1 is located.

Also, the hygienising means 6 of the hygienising apparatus 1 can comprise a second tank 27, arranged for containing a washing product, for example a detergent product.

The second tank 27 can be arranged above said first tank 24. The hygienising means 6 of the hygienising apparatus 1 can further comprise a third tank 28, arranged for containing a further washing product, for example a polishing product.

The third tank 28 can be arranged above said second tank 27. The hygienising means 6 of the hygienising apparatus 1 can further comprise a fourth tank 29, arranged for containing part of the washing fluid after the start of the washing cycle. Part of the washing fluid used in the washing cycle is in fact recovered and stored in the fourth tank 29 and is then subsequently reused in the continuation of the washing cycle.

The fourth tank 29 can be arranged below said first tank 24.

The first tank 24, the second tank 27, the third tank 28 and the fourth tank 29 can have a substantially parallelpipedon shape.

The first tank 24, the second tank 27, the third tank 28 and the fourth tank 29 can be suitably insulated to maximise the energy yield of the hygienising apparatus 1.

The first tank 24 and the fourth tank 29 can each comprise an indicator of the water level and of the washing fluid, respectively.

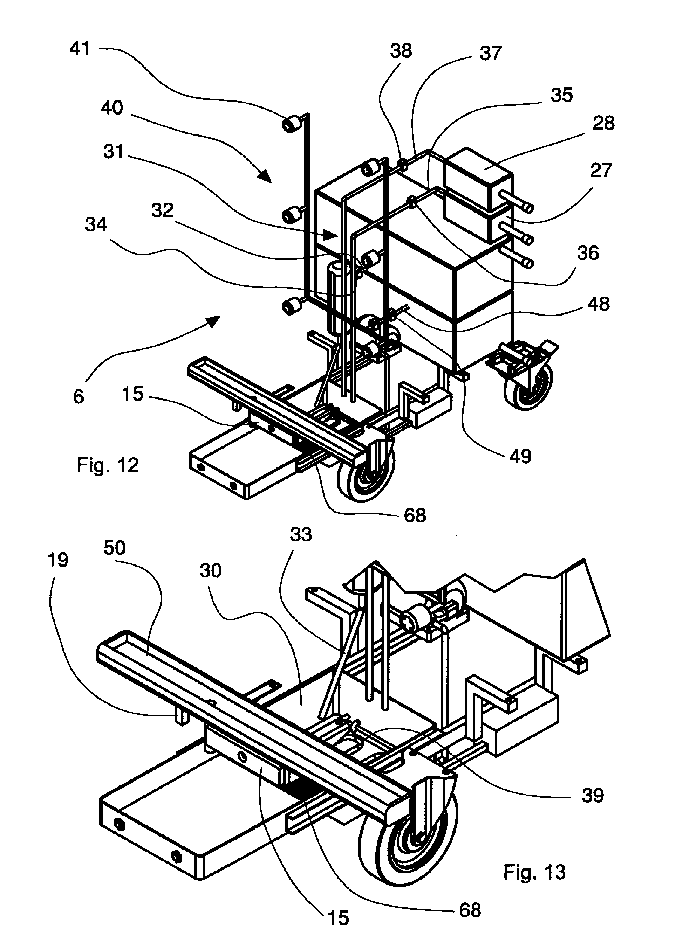

The hygienising apparatus 1 further comprises a collecting and mixing tank 30, shown, in particular, in Figure 13, arranged for collecting the water coming from the first tank 24, the detergent product coming from the second tank 27, and the polishing product coming from the third tank 28 and thus obtaining a washing fluid.

The collecting and mixing tank 30 is mounted on guides in such a manner as to be able to be removed from the hygienising apparatus 1 to be emptied and/or cleaned by an operator.

The collecting and mixing tank 30 is provided with a heating element 39 arranged for heating the washing fluid to be then used in the washing cycle.

The heating element 39 can comprise a resistance.

The collecting and mixing tank 30 further comprises filtering means 68, comprising, for example, a grid or a network arranged transversely in said collecting and mixing tank 30 to retain possible solid particles found in the washing water coming from the thermal conditioning station 2.

The filtering means 68 can further comprise filters suitable for enabling substances to be eliminated that are present in the washing water coming from the thermal conditioning station 2 that can generate unpleasant odours, such as residues of chlorination, limescale and heavy metals such as lead and/or copper.

Further, the hygienising apparatus 1 can comprise a water softening device 25 arranged for decreasing the hardness of the water of the mains water supply, preventing the formation of limescale in the hygienising apparatus 1.

In order to enable the components that will form the washing fluid and the washing fluid to pass through, conduit means 31 is provided.

In particular, the conduit means 31 comprises a first conduit 32 that connects the first tank 24 to the water softening device 25 and a second conduit 33 that connects the water softening device 25 to the collecting and mixing tank 30. In this manner, the water coming from the mains water supply can reach the collecting and mixing tank 30. In particular, the water drops by gravity into the first conduit 32 and into the second conduit 33.

In the first conduit 32 a first adjusting valve 34 is provided that is arranged for adjusting the quantity of water exiting the first tank 24.

Alternatively, the water coming from the mains water supply can reach the collecting and mixing tank 30 through the use of a dosing pump.

The conduit means 31 further comprises a third conduit 35 that connects the second tank 27 to the collecting and mixing tank 30. In this manner, the detergent product can reach the collecting and mixing tank 30, the detergent product also descending, in particular, by gravity into the third conduit 35.

In the third conduit 35 a second adjusting valve 36 is provided that is arranged for adjusting the quantity of detergent product exiting second tank 27.

Alternatively, the detergent product can reach the collecting and mixing tank 30 using a dosing pump.

The conduit means 31 further comprises a fourth conduit 37 that connects the third tank 28 to the collecting and mixing tank 30. In this manner, the polishing product can reach the collecting and mixing tank 30, the polishing product also descending, in particular, by gravity into the fourth conduit 37.

In the fourth conduit 37 a third adjusting valve 38 is provided that is arranged for adjusting the quantity of polishing product exiting the third tank 28.

Alternatively, the polishing product can reach the collecting and mixing tank 30 using a dosing pump.

The hygienising means 6 of the hygienising apparatus 1 further comprises spraying means 40, arranged for spraying the washing fluid toward the thermal conditioning station 2. The spraying means 40 comprises a plurality of nozzles 41, which can be distributed uniformly or in groups, each nozzle being able to direct the spray of washing fluid in a set direction toward the thermal conditioning station 2.

The nozzles 41 extend from a separating wall 42, shown for example in Figure 9, arranged inside the chamber 5, toward the thermal conditioning station 2, when the latter and the hygienising apparatus 1 are coupled.

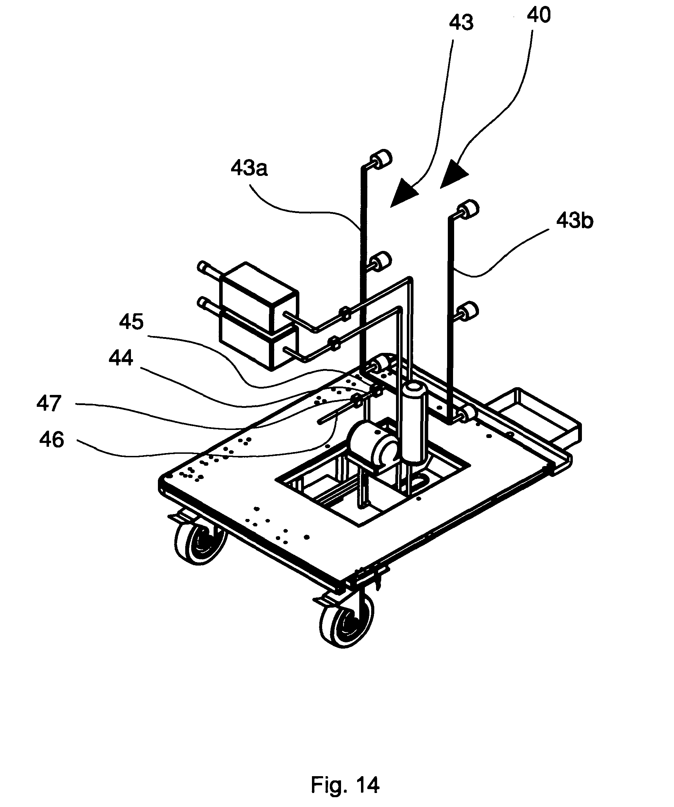

The separating wall 42 divides the chamber 5 into a first part 5a and into a second part 5b and enables it to be prevented that a large part of the washing fluid sprayed by the nozzles 41 in the second part 5b toward the thermal conditioning station 2 can fall back into the first part 5a. The nozzles 41 are mutually connected by a plurality of tubes 43 that can comprise, for example, a first tube 43a and a second tube 43b, as illustrated, for example, in Figure 14. The first tube 43a and the second tube 43b converge on a fifth conduit 44 that connects the nozzles 41 to the collecting and mixing tank 30.

In the fifth conduit 44 a fourth adjusting valve 45 is provided that is arranged for adjusting the quantity of washing fluid that has to exit the collecting and mixing tank 30 to reach the nozzles 41.

The fourth tank 29 is connected to the collecting and mixing tank 30 by a sixth conduit 46 that can lead into the fifth conduit 44 and thus into the aforesaid tank.

In the sixth conduit 46 a fifth adjusting valve 47 is provided that is arranged for adjusting the quantity of washing fluid already used in the washing cycle that has to exit the collecting and mixing tank 30 to be collected in the fourth tank 29.

The fourth tank 29 is further connected to the plurality of tubes 43 by a seventh conduit 48, shown, in particular, in Figure 12.

In the seventh conduit 48 a sixth adjusting valve 49 is provided that is arranged for adjusting the quantity of washing fluid already used in the washing cycle and collected in the fourth tank 29 that has to reach the nozzles 41 to be further used in the washing cycle.

The spraying means 40 can comprise, in addition or alternatively to the nozzles 41, rotating nozzles, which are not illustrated, for example of the whirling type.

Part of the washing fluid, sprayed by the spraying means 40, does not reach the thermal conditioning station 2, but hits the sealing means 21. This part of washing fluid can be recovered by collecting means that conveys the part toward the collecting and mixing tank 30.

The collecting means can comprise a gutter channel 50 arranged below the rear wall 8 and having a length that is such as to enable the washing fluid falling from the sealing means 21 to be collected.

In this manner, owing to the collecting means, to the collecting and mixing tank 30 and to the fourth tank 29 it is possible to recycle part of the washing fluid already used in the washing cycle.

The hygienising means 6 of the hygienising apparatus 1 further comprises pumping means 26, which can comprise a pump arranged for pressurising the washing fluid and part of the washing fluid already used to make it flow into the conduit means 31 and into the plurality of tubes 43.

The hygienising apparatus 1 further comprises a control panel 51, arranged on the front wall 7, in such a manner that the operator can access it easily when he has to start the hygienising cycle.

The hygienising apparatus 1 further comprises an electrical supply 52, which comprises a supply cable to be connected to an electric system.

In order to run the sanitising cycle, the hygienising apparatus 1 comprises sanitising means 53 that is suitable for sanitising the thermal conditioning station 2.

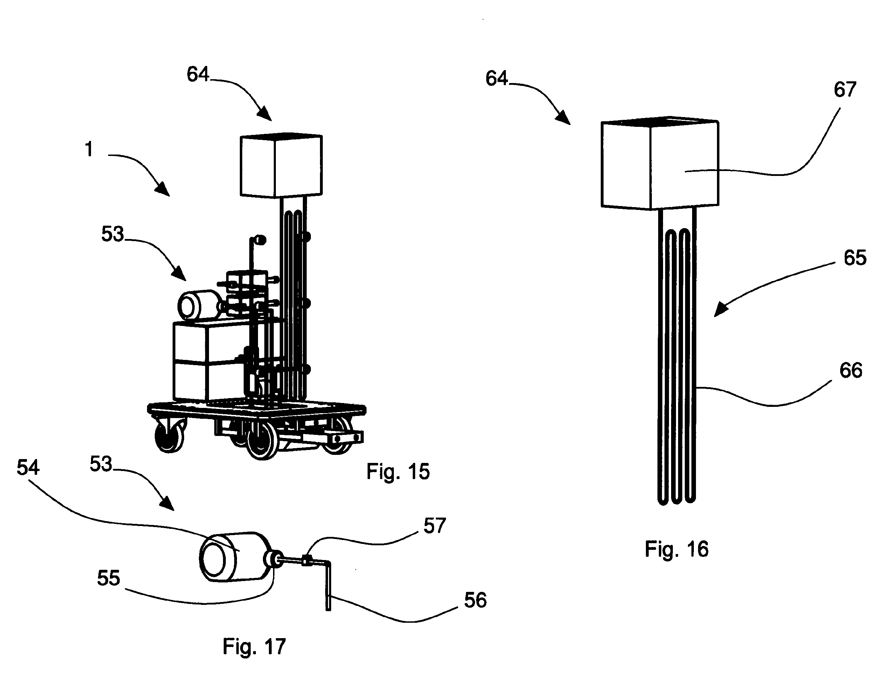

In a first embodiment, illustrated in Figures 15 to 17, the sanitising means 53 comprises a fifth tank 54 intended for containing a bactericide agent, such as pressurised nitrogen. The fifth tank 54 can be a pressurised cylinder.

The sanitising means 53 further comprises a pressure reducing device 55 intended for decreasing the pressure of the nitrogen so that it can be used in the sanitising cycle.

The nitrogen is introduced into the internal space 20 of the thermal conditioning station 2 via the nozzles 41. In use, the internal space 20 is saturated with nitrogen.

The fifth tank 54 is in fact connected to the plurality of tubes 43 and thus to the nozzles 41, via an eighth conduit 56.

The eighth conduit 56 is provided with a first solenoid valve 57, used for connecting the fifth tank 54 to the nozzles 41 during the sanitising cycle.

In this first embodiment, the sanitising cycle occurs after the washing cycle.

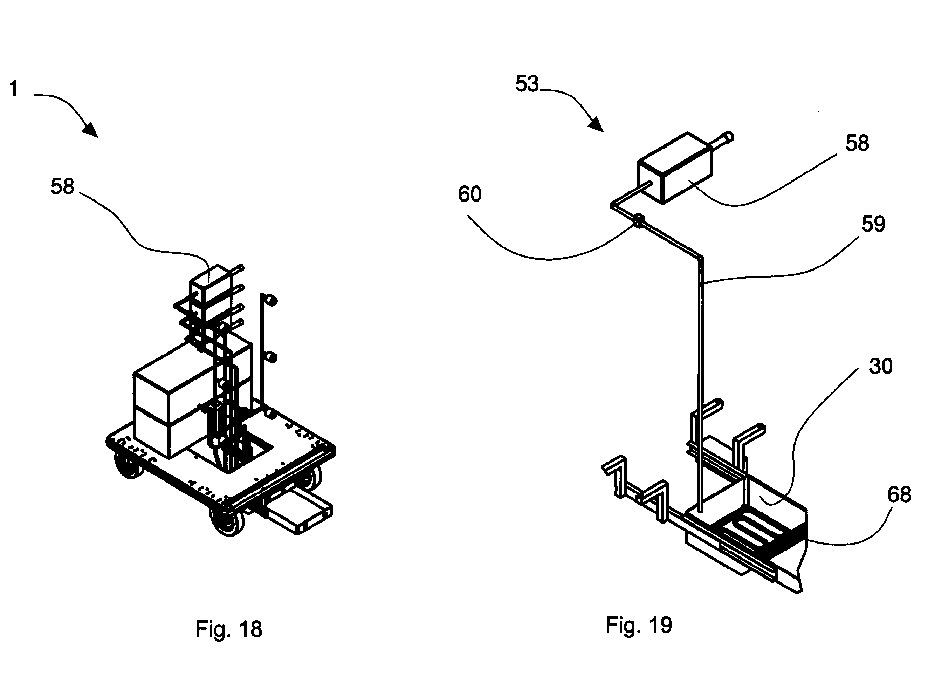

In a second embodiment shown in Figures 18 and 19, the sanitising means 53 comprises a sixth tank 58, intended for containing a chemical sanitising product.

The sixth tank 58 can be located above the third tank 28.

The fifth tank 58 can have a substantially parallelpipedon shape.

This chemical sanitising product is added to the washing fluid before the end of the washing cycle.

Consequently, in this second embodiment, the sanitising cycle occurs at the same time as the washing cycle.

For this purpose, the sixth tank 58 is connected to the collecting and mixing tank 30 via a ninth conduit 59.

The ninth conduit 59 is provided with a sixth adjusting valve 60 that is arranged for adjusting the quantity of chemical sanitising product to be added to the washing fluid.

The chemical sanitising product descends by gravity into the collecting and mixing tank 30 once the sixth adjusting valve 60 has been opened.

Alternatively, the chemical sanitising product can reach the collecting and mixing tank 30 using a dosing pump.

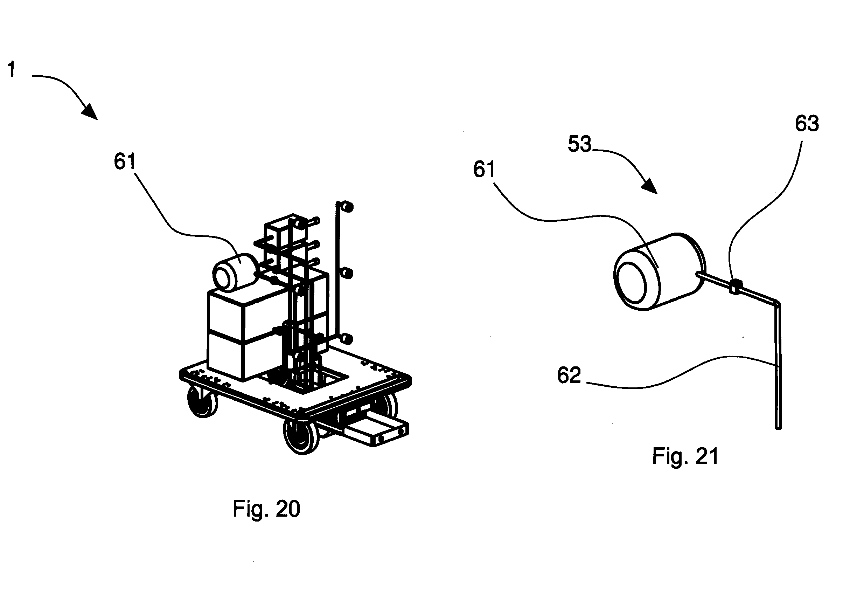

In a third embodiment, illustrated in Figures 20 and 21, the sanitising means 53 comprises a steam generating device 61 intended for generating steam to be used as a sanitising agent.

The steam generating device 61 can remove water from the first tank 24 or from an auxiliary tank, which is not shown. The steam is introduced into the internal space 20 of the thermal conditioning station 2 via the nozzles 41.

The steam generating device 61 is in fact connected to the plurality of tubes 43 and thus to the nozzles 41, via a tenth conduit 62.

The tenth conduit 62 is provided with a second solenoid valve 63, used for connecting the steam generating device 61 to the nozzles 41 during the sanitising cycle.

In this third embodiment, the sanitising cycle occurs after the washing cycle.

In a fourth embodiment, which is not illustrated, the conditioning means of the thermal conditioning station 2 acts as sanitising means 53. The resistances and the fans of the thermal conditioning station 2 are in fact activated to generate flows of hot air that are able to heat even the cold chamber that is not provided with resistances.

Also in this fourth embodiment, the sanitising cycle occurs after the washing cycle.

In order to run the drying cycle, the hygienising apparatus 1 comprises a refrigerating unit 64, shown in Figures 15 and 16.

In Figure 15, in particular, the refrigerating unit 64 is shown in association with the first embodiment of the sanitising means 53, but can also be associated with all the other embodiments of the sanitising means disclosed above. The refrigerating unit 64 has the object of condensing the water vapour present in the internal space 20 of the thermal conditioning station 2 and in the second part 5b after the washing cycle and the sanitising cycle.

The refrigerating unit 64 is located in the second part 5b. The refrigerating unit 64 comprises an evaporating element 65 that can be provided with a coil 66 that extends substantially over the entire width and height of the hygienising apparatus 1.

A coolant fluid is made to flow into the coil 66 so that a surface of the latter has a temperature that is lower than the temperature of the air present in the second part 5b of the hygienising apparatus 1 and in the internal space 20 of the thermal conditioning station 2.

The location and configuration of the evaporating element 65 are such as to enable it to be touched by the air present inside the second part 5b and inside the internal space 20. The air is saturated with the water vapour produced during the washing and/or sanitising cycle, which water vapour, touching the surface of the coil 66, which is at a temperature that is lower than the air, condenses and falls into a collecting container, which is not shown, which is positioned in the place of the collecting and mixing tank 30 during the drying step.

The air containing the water vapour can be circulated by the fans of the thermal conditioning station 2 to improve the drying cycle, making it faster.

In order to make the drying cycle more effective, the hygienising apparatus 1 can be provided with a condensing unit 67.

The management and control unit and the further management and control unit coordinate the operation of the hygienising cycle.

The operation of the hygienising apparatus 1 according to the invention will be disclosed below. In particular, a hygienising cycle will be disclosed, comprising at least one washing cycle, at least one sanitising cycle and/or at least one drying cycle.

Before activating the washing cycle, it is necessary to perform some preliminary operations.

The fourth tank 29 is removed from the hygienising apparatus 1 and the contents thereof, i.e. the residue of the previous washing cycle, is evacuated.

Also the collecting and mixing tank 30 is removed from the hygienising apparatus 1 by the guides thereof to be cleaned together with the filtering means 68, in order to eliminate possible solid residues retained during the preceding washing cycle.

The first adjusting valve 34 is taken to an open configuration to enable cleaning water coming from the mains water supply to enter the first tank 24.

The second tank 27, the third tank 28 and the sixth tank 58, if it is present, are topped up.

If present, fifth tank 54 and, in particular, the nitrogen pressure contained therein, are checked and possibly replaced.

At the end of the preliminary operations, the hygienising apparatus 1 is coupled with the thermal conditioning station 2.

In order to perform coupling, the thermal conditioning station 2 has to be activated, for example by pressing a key on a control panel thereof, and the thermal conditioning station 2 has to be taken to a position where at least one door of the thermal conditioning station 2 arranged on the coupling side is open.

At this point an operator can move the hygienising apparatus 1 near the thermal conditioning station 2, making the aligning means and the further aligning means meet, such as to activate the electromagnet 16 to exert a force on the block 15 that is sufficient to retain the hygienising apparatus 1 coupled with the thermal conditioning station 2. Owing to the sealing means 21, a hermetic seal is further created in the contact perimeter between the hygienising apparatus 1 and the thermal conditioning station 2.

The collecting and mixing tank 30 is then place on the guides and is slid by a mechanical actuator.

The recognition means is activated and the thermal conditioning station 2 recognises the hygienising apparatus 1 as such, this enabling the conditioning means not to be activated in an inappropriate manner.

Once the hygienising apparatus 1 is coupled with the thermal conditioning station 2, the hygienising apparatus is connected by the operator to the electric system via the supply cable and is activated, for example by pressing a key on the control panel.

A further key provided on the control panel starts the washing cycle.

The washing cycle provides the following steps:
- filling the collecting and mixing tank 30 with water and starting up the heating element 39;
- adding detergent product to the water being heated in the collecting and mixing tank 30 to obtain a heated solution;
- circulating the heated solution and spraying the heated solution via the spraying means 40 in the thermal conditioning station 2. The temperature of the heated solution can be comprised between 60 and 80°C and the washing time by the heated solution can be comprised between 2 and 4 minutes or at least be a sufficient time to guarantee correct washing;
- discharging waste water in the fourth tank 29;
- filling the collecting and mixing tank 30 with water and starting up the heating element 39;
- continuously rinsing the thermal conditioning station 2 with the heated solution;
- filling the collecting and mixing tank 30 with water and starting the heating element 39,
- adding polishing product to the water being heated or in the collecting and mixing tank 30;
- rinsing continuously the thermal conditioning station 2 with heated solution;
- possibly activating, via a wireless command generated by the hygienising apparatus 1 on the thermal conditioning station 2, the conditioning means of the thermal conditioning station 2 and, in particular the fans thereof, to generate flows of air and make the washing cycle more effective.

The washing and/or rinsing steps can be repeated several times.

The sanitising cycle differs according to the sanitising means 53 present in the hygienising apparatus 1.

In the case of the first embodiment of the sanitising means 53, after the washing cycle, nitrogen is removed from the fifth tank 54 in such a quantity as to saturate the internal space 20 of the thermal conditioning station 2. Using high-concentration nitrogen, disinfections is obtained.

The time required to perform this sanitisation is about 1 hour, or any way a time that is sufficient to ensure sanitisation.

In the case of the second embodiment of the sanitising means 53, from the third tank 28 a sanitising product is removed and added to water in a further washing step preceding the rinsing of the thermal conditioning station 2.

In the case of the third embodiment of the sanitising means 53, after the washing cycle, the steam generating device 61 is activated to generate dry steam to be introduced into the internal space 20 of the thermal conditioning station 2 through the nozzles 41 used during the washing cycle. The second solenoid valve 63 supplies the nozzles 41 with the steam exiting the steam generating device 61 through the pressure difference.

The time necessary for performing this sanitisation is about 10-15 minutes or anyway a time that is sufficient for ensuring sanitisation.

In the case of the fourth embodiment of the sanitising means 53, the resistances and fans are activated on the hot side of the thermal conditioning station 2, whilst the hygienising apparatus 1 is still connected, in such a manner as to enable also the cold space of the thermal conditioning station 2 to be heated that is not provided with resistances.

The time necessary for performing this sanitisation is about 10 minutes, or any way sufficient time to ensure sanitisation, and the air temperature is taken to about 90°C. Subsequently a drying cycle can be provided, achieved through the interaction between the conditioning means of the thermal conditioning station 2 and the condensing unit 67 of the hygienising apparatus 1.

In fact, owing to the wireless connection between the management and control unit of the hygienising apparatus 1 and the further management and control unit of the thermal conditioning station 2, it is possible to activate substantially simultaneously the fans and the electrical resistances of the thermal conditioning station 2 to evaporate the water present and the condensing unit 67, if present.

Owing to the hygienising apparatus 1, the hygienising cycle of the thermal conditioning station 2 is thorough, fast an automatic.

Further, the hygienising apparatus 1 enables leaks of fluid or steam into the external environment to be avoided, and it can thus be installed in any type of environment, including a ward of a hospital facility.

In this manner, the need to set up equipped rooms for hygienising the thermal conditioning station 2 is avoided. Also, the hygienising apparatus 1, according to the invention, enables the use of manpower to be reduced, inasmuch as the hygienising cycle occurs in the same place in which the thermal conditioning station 2 is located to perform the conditioning operations on a carriage and the thermal conditioning station 2 does not have to be converted to a hygienising room. This further avoids the need for an additional thermal conditioning station 2 to replace the thermal conditioning station 2 when it is subjected to hygienising cycles.

## Claims

1. Mobile hygienising apparatus (1) designed to be coupled with a thermal conditioning station (2), said thermal conditioning station (2) being provided with an internal space (20), said hygienising apparatus comprising a box body (3) defining a chamber (5) in which hygienising means (6) is housed that is arranged for performing a hygienising cycle in said internal space (20) of said thermal conditioning station (2) **characterised in that** said hygienising apparatus (1) is further provided with coupling means (13) and with sealing means (21) designed to permit a seal connection with said thermal conditioning station (2), in such a manner that said chamber (5) is sealingly connected to said internal space (20).

2. Hygienising apparatus (1) according to claim 1, wherein said coupling means (13) comprises a block (15), made of a ferromagnetic material, arranged for interacting with an electromagnet (16) situated on said thermal conditioning station (2), or comprises an electromagnet (16), arranged for interacting with a block (15) made of a ferromagnetic material situated on said thermal conditioning station (2), in order to make said seal connection.

3. Hygienising apparatus (1) according to any preceding claim, wherein said sealing means (21) is arranged for interacting with corresponding further sealing means (22) arranged on a perimeter of a front face (23) of said thermal conditioning station (2) such as to ensure an air and water seal between said hygienising apparatus (1) and said thermal conditioning station (2).

4. Hygienising apparatus (1) according to any preceding claim, wherein said sealing means (21) comprises a seal or a plurality of seals, arranged on a perimeter of a rear wall (8) of said box body (3) intended for contacting said thermal conditioning station (2).

5. Hygienising apparatus (1) according to any preceding claim, wherein said hygienising means (6) comprises a first tank (24) arranged for containing water to be used in a washing cycle of said hygienising cycle to form a washing fluid, said first tank (24) being provided with a connecting element for connecting to the mains water supply of a place in which said hygienising apparatus (1) is placed.

6. Hygienising apparatus (1) according to any preceding claim, wherein said hygienising means (6) comprises a second tank (27), arranged for containing a washing product to be used to form a washing fluid, said washing product being a detergent product.

7. Hygienising apparatus (1) according to any preceding claim, wherein said hygienising means (6) comprises a third tank (28), arranged for containing a further washing product to be used to form a washing fluid, said further washing product being a polishing product.

8. Hygienising apparatus (1) according to claim 6, or 7, wherein said hygienising means (6) comprises a fourth tank (29), arranged for containing and recycling said washing fluid after the start of a washing cycle, and a collecting and mixing tank (30), arranged for collecting said water coming from said first tank (24), said detergent product coming from said second tank (27), and/or said polishing product coming from said third tank (28) and thus obtaining said washing fluid.

9. Hygienising apparatus (1) according to claim 8, wherein said collecting and mixing tank (30) is mounted on guides in such a manner as to be able to be removed from said hygienising apparatus (1) to be emptied and/or cleaned, said mixing tank being able to be further provided with a heating element (39) arranged for heating said washing fluid and/or filtering means (68), arranged for performing a filtering operation on the washing water coming from said thermal conditioning station (2).

10. Hygienising apparatus (1) according to any one of claims 5 to 9, wherein said hygienising means (6) further comprises spraying means (40), arranged for spraying said washing fluid to said thermal conditioning station (2), said spraying means being able to comprise a plurality of nozzles (41), each nozzle being able to direct a spray of said washing fluid in a set direction to said thermal conditioning station (2).

11. Hygienising apparatus (1) according to any one of claims 4 to 10, and further comprising collecting means arranged below said rear wall (8) and having a length that is such as to enable part of said washing fluid falling from said sealing means (21) to be collected and conveyed to said collecting and mixing tank (30), and/or sanitising means (53) suitable for sending a sanitising fluid into said thermal conditioning station (2), said sanitising means (53) being able to comprise a fifth tank (54) intended for containing a bactericide agent.

12. Hygienising apparatus (1) according to claim 11, wherein said fifth tank (54) is connected to said spraying means (40).

13. Hygienising apparatus (1) according to claim 11, or 12, wherein said sanitising means (53) comprises a sixth tank (58) intended for containing a chemical sanitising product, said sixth tank (58) being able to be connected to said spraying means (40).

14. Hygienising apparatus (1) according to any one of claims 11 to 13 wherein said sanitising means (53) comprises a steam generating device (61) intended for generating steam, said steam generating device being able to be connected to said spraying means (40).

15. Hygienising apparatus (1) according to any preceding claim, and further comprising a refrigerating unit (64) intended for condensing water vapour present in an internal space (20) of said thermal conditioning station (2) and in said chamber (5), said refrigerating unit (64) comprising an evaporating element (65) provided with a coil (66) that extends substantially for the entire width and height of said hygienising apparatus (1) and is suitable for making a refrigerating fluid flow inside that is such as to take a surface of said coil (66) to a temperature that is lower than the temperature of the air in said chamber (5) and in said internal space (20) of said thermal conditioning station (2).

16. Hygienising apparatus (1) according to any preceding claim, and further comprising, recognition means arranged such said thermal conditioning station (2) recognises said hygienising apparatus (1), said recognition means comprising an abutting element (19) suitable for interacting with a microswitch (19a) arranged on said thermal conditioning station (2), or a RFID device that is read by a reading unit of said thermal conditioning station (2).

17. Hygienising apparatus (1) according to claim 16, further comprising a management and control unit suitable for exchanging signals with a further management and control unit of said thermal conditioning station (2).

## Patentansprüche

1. Mobile Hygienisierungsapparatur (1), die ausgestaltet ist, um mit einer Station (2) zur thermalen Konditionierung gekoppelt zu werden, wobei die Station (2) zur thermalen Konditionierung mit einem Innenraum (20) versehen ist, und wobei die Hygienisierungsapparatur einen kastenartigen Körper (3) aufweist, der eine Kammer (5) definiert, in der ein Hygienisierungsmittel (6) untergebracht ist, das eingerichtet ist, um einen Hygienisierungszyklus in dem Innenraum (20) der Station (2) zur thermalen Konditionierung durchzuführen, **dadurch gekennzeichnet, dass** die Hygienisierungsapparatur (1) ferner mit Kopplungsmitteln (13) und mit Dichtungsmitteln (21) versehen ist, die ausgestaltet sind, um eine dichte Verbindung mit der Station (2) zur thermalen Konditionierung auf solche Weise zu ermöglichen, dass die Kammer (5) dicht mit dem Innenraum (20) verbunden ist.

2. Hygienisierungsapparatur (1) gemäß Anspruch 1, wobei das Kopplungsmittel (13) einen Block (15) aufweist, der aus einem ferromagnetischen Material gebildet ist, und der eingerichtet ist, um mit einem Elektromagneten (16) zu interagieren, der auf der Station (2) zur thermalen Konditionierung angeordnet ist, oder das einen Elektromagneten (16) aufweist, der eingerichtet ist, um mit einem Block (15) zu interagieren, der aus einem ferromagnetischen Material gebildet ist, und auf der Station (2) zur thermalen Konditionierung angeordnet ist, um die dichte Verbindung zu bilden.

3. Hygienisierungsapparatur (1) gemäß einem der vorherigen Ansprüche, wobei die Dichtungsmittel (21) eingerichtet sind, um mit korrespondierenden weiteren Dichtungsmitteln (22) zu interagieren, die auf einem Umfang einer Vorderseite (23) der Station (2) zur thermalen Konditionierung derart eingerichtet sind, um eine Luft- und Wasserabdichtung zwischen der Hygienisierungsapparatur (1) und der Station (2) zur thermalen Konditionierung sicher zu stellen.

4. Hygienisierungsapparatur (1) gemäß einem der vorherigen Ansprüche, wobei die Dichtungsmittel (21) eine Dichtung oder eine Vielzahl von Dichtungen aufweisen, die auf einem Umfang einer Rückwand (8) des kastenartigen Körpers (3) eingerichtet sind, der für das Kontaktieren der Station (2) zur thermalen Konditionierung vorgesehen ist.

5. Hygienisierungsapparatur (1) gemäß einem der vorherigen Ansprüche, wobei das Hygienisierungsmittel (6) einen ersten Tank (24) aufweist, der eingerichtet ist, um Wasser zu enthalten, das in einem Waschzyklus des Hygienisierungszyklus verwendet wird, um eine Waschflüssigkeit zu bilden, wobei der erste Tank (24) mit einem verbindenden Element versehen ist, um eine Verbindung zu der Hauptwasserversorgung einer Stelle zu schaffen, an der die Hygienisierungsapparatur (1) platziert ist.

6. Hygienisierungsapparatur (1) gemäß einem der vorherigen Ansprüche, wobei das Hygienisierungsmittel (6) einen zweiten Tank (27) aufweist, der eingerichtet ist, um ein Waschprodukt zu enthalten, das dazu verwendet wird, um eine Waschflüssigkeit zu bilden, wobei das Waschprodukt ein Detergenzprodukt ist.

7. Hygienisierungsapparatur (1) gemäß einem der vorherigen Ansprüche, wobei das Hygienisierungsmittel (6) einen dritten Tank (28) aufweist, der eingerichtet ist, um ein weiteres Waschprodukt zu enthalten, das dazu verwendet wird, um eine Waschflüssigkeit zu bilden, wobei das weitere Waschprodukt ein Politurprodukt ist.

8. Hygienisierungsapparatur (1) gemäß Anspruch 6 oder 7, wobei das Hygienisierungsmittel (6) einen vierten Tank (29) aufweist, der eingerichtet ist, um die Waschflüssigkeit zu enthalten und nach dem Beginn eines Waschzyklus zu recyceln, und einen Sammel- und Mischtank (30) aufweist, der eingerichtet ist, um das Wasser, das von dem ersten Tank (24) kommt, das Detergenzprodukt, das von dem zweiten Tank (27) kommt, und/oder das Politurprodukt, das von dem dritten Tank (28) kommt, einzusammeln, und so die Waschflüssigkeit zu erhalten.

9. Hygienisierungsapparatur (1) gemäß Anspruch 8, wobei der Sammel- und Mischtank (30) auf Führungen auf solch eine Weise montiert ist, um von der Hygienisierungsapparatur (1) zum Entleeren und Reinigen entfernt zu werden, wobei der Mischtank dazu vorgesehen ist, um ferner mit einem Heizelement (39) versehen zu werden, das eingerichtet ist, um die Waschflüssigkeit zu erwärmen, und/oder mit Filtermitteln (68) versehen zu werden, die dazu eingerichtet sind, um einen Filtervorgang an dem Wasser durchzuführen, das von der Station (2) zur thermalen Konditionierung kommt.

10. Hygienisierungsapparatur (1) gemäß einem der Ansprüche 5 bis 9, wobei das Hygienisierungsmittel (6) ferner Sprühmittel (40) aufweist, die dazu eingerichtet sind, um die Waschflüssigkeit zu der Station (2) zur thermalen Konditionierung zu sprühen, wobei die Sprühmittel in der Lage sind, eine Vielzahl von Düsen (41) aufzuweisen, wobei jede Düse in der Lage ist, einen Spray der Waschflüssigkeit in eine gegebene Richtung zu der Station (2) zur thermalen Konditionierung zu richten.

11. Hygienisierungsapparatur (1) gemäß einem der Ansprüche 4 bis 10, die ferner Sammelmittel aufweist, die unterhalb der Rückwand (8) eingerichtet sind und eine Länge aufweisen, die derart ausgebildet ist, um es einem Teil der Waschflüssigkeit, die von den Dichtungsmitteln (21) fällt, zu ermöglichen, gesammelt und zu dem Sammel- und Mischtank (30) befördert zu werden, und/oder Reinigungsmittel (53) aufweist, die geeignet sind, um eine Reinigungsflüssigkeit in die Station (2) zur thermalen Konditionierung zu senden, wobei die Reinigungsmittel (53) in der Lage sind, einen fünften Tank (54) aufzuweisen, der vorgesehen ist, um ein bakterizides Agens zu enthalten.

12. Hygienisierungsapparatur (1) gemäß Anspruch 11, wobei der fünfte Tank (54) mit den Sprühmitteln (40) verbunden ist.

13. Hygienisierungsapparatur (1) gemäß Anspruch 11 oder 12, wobei die Reinigungsmittel (53) einen sechsten Tank (58) aufweisen, der dazu vorgesehen ist, um ein chemisches Reinigungsprodukt zu enthalten, wobei der sechste Tank (58) in der Lage ist, mit den Sprühmitteln (40) verbunden zu werden.

14. Hygienisierungsapparatur (1) gemäß einem der Ansprüche 11 bis 13, wobei die Reinigungsmittel (53) eine dampferzeugende Vorrichtung (61) aufweisen, die dazu vorgesehen ist, um Dampf zu erzeugen, wobei die dampferzeugende Vorrichtung in der Lage ist, mit den Sprühmitteln (40) verbunden zu werden.

15. Hygienisierungsapparatur (1) gemäß einem der vorherigen Ansprüche, die ferner eine Kühleinheit (64) aufweist, die dazu vorgesehen ist, um Wasserdampf zu kondensieren, der in einem Innenraum (20) der Station (2) zur thermalen Konditionierung und in der Kammer (5) vorhanden ist, wobei die Kühleinheit (64) ein Verdampfungselement (65) aufweist, das mit einer Spule (66) versehen ist, die sich im Wesentlichen über die gesamte Breite und Höhe der Hygienisierungsapparatur (1) erstreckt und in der Lage ist, einen Kühlflüssigkeitsfluss im Inneren zu erzeugen, der derart ausgestaltet ist, um eine Fläche der Spule (66) auf eine Temperatur zu bringen, die niedriger ist, als die Temperatur der Luft in der Kammer (5) und in dem Innenraum (20) der Station (2) zur thermalen Konditionierung.

16. Hygienisierungsapparatur (1) gemäß einem der vorherigen Ansprüche, die ferner Erkennungsmittel aufweist, die derart eingerichtet sind, dass die Station (2) zur thermalen Konditionierung die Hygienisierungsapparatur (1) erkennt, wobei die Erkennungsmittel ein Angrenzelement (19) aufweisen, das geeignet ist, um mit einem Mikroschalter (19a) zu interagieren, der auf der Station (2) zur thermalen Konditionierung eingerichtet ist, oder eine RFID-Vorrichtung, die von einer Leseeinheit der Station (2) zur thermalen Konditionierung gelesen wird.

17. Hygienisierungsapparatur (1) gemäß Anspruch 16, die ferner eine Verwaltungs- und Steuerungseinheit aufweist, die geeignet ist, um Signale mit einer weiteren Verwaltungs- und Steuerungseinheit der Station (2) zur thermalen Konditionierung auszutauschen.

## Revendications

1. Appareil mobile d'hygiénisation (1) conçu pour être couplé à une station de conditionnement thermique (2), ladite station de conditionnement thermique (2) étant prévue avec un espace interne (20), ledit appareil d'hygiénisation comprenant un corps de boîte (3) définissant une chambre (5) dans laquelle sont logés des moyens d'hygiénisation (6) qui sont organisés pour effectuer un cycle d'hygiénisation dans ledit espace interne (20) de ladite station de conditionnement thermique (2), **caractérisé en ce que** ledit appareil d'hygiénisation (1) est en outre muni de moyens de couplage (13) et de moyens d'étanchéité (21) conçus pour permettre un raccordement étanche avec ladite station de conditionnement thermique (2), d'une manière telle que ladite chambre (5) est connectée de manière étanche audit espace interne (20).

2. Appareil d'hygiénisation (1) selon la revendication 1, dans lequel lesdits moyens de couplage (13) comprennent un bloc (15), réalisé en un matériau ferromagnétique, organisé pour interagir avec un électro-aimant (16) situé sur ladite station de conditionnement thermique (2), ou comprennent un électro-aimant (16), organisé pour interagir avec un bloc (15) réalisé en un matériau ferromagnétique et situé sur ladite station de conditionnement thermique (2), afin de former ledit raccordement étanche.

3. Appareil d'hygiénisation (1) selon une quelconque revendication précédente, dans lequel lesdits moyens d'étanchéité (21) sont organisés pour interagir avec des moyens d'étanchéité supplémentaires (22) correspondants disposés sur un périmètre d'une face avant (23) de ladite station de conditionnement thermique (2) de manière à garantir une jonction étanche à l'air et à l'eau entre ledit appareil d'hygiénisation (1) et ladite station de conditionnement thermique (2).

4. Appareil d'hygiénisation (1) selon une quelconque revendication précédente, dans lequel lesdits moyens d'étanchéité (21) comprennent un joint étanche ou une pluralité de joints étanches, disposé(s) sur un périmètre d'une paroi arrière (8) dudit corps de boîte (3) destiné à être en contact avec ladite station de conditionnement thermique (2).

5. Appareil d'hygiénisation (1) selon une quelconque revendication précédente, dans lequel lesdits moyens d'hygiénisation (6) comprennent un premier réservoir (24) organisé pour contenir de l'eau destinée à être utilisée dans un cycle de lavage dudit cycle d'hygiénisation pour constituer un fluide de lavage, ledit premier réservoir (24) étant muni d'un élément de raccordement pour le raccordement à l'alimentation principale en eau d'un site dans lequel ledit appareil d'hygiénisation (1) est mis en place.

6. Appareil d'hygiénisation (1) selon une quelconque revendication précédente, dans lequel lesdits moyens d'hygiénisation (6) comprennent un deuxième réservoir (27), organisé pour contenir un produit de lavage destiné à être utilisé pour former un fluide de lavage, ledit produit de lavage étant un produit détergent.

7. Appareil d'hygiénisation (1) selon une quelconque revendication précédente, dans lequel lesdits moyens d'hygiénisation (6) comprennent un troisième réservoir (28), organisé pour contenir un produit de lavage supplémentaire destiné à être utilisé pour former un fluide de lavage, ledit produit de lavage supplémentaire étant un produit lustrant.

8. Appareil d'hygiénisation (1) selon la revendication 6 ou 7, dans lequel lesdits moyens d'hygiénisation (6) comprennent un quatrième réservoir (29), organisé pour contenir et recycler ledit produit de lavage après le démarrage d'un cycle de lavage, et un réservoir de collecte et de mélange (30), organisé pour collecter ladite eau provenant dudit premier réservoir (24), ledit produit détergent provenant dudit deuxième réservoir (27), et/ou ledit produit lustrant provenant dudit troisième réservoir (28), et fournissant ainsi ledit fluide de lavage.

9. Appareil d'hygiénisation (1) selon la revendication 8, dans lequel ledit réservoir de collecte et de mélange (30) est monté sur des guides d'une manière telle qu'il est apte à être retiré dudit appareil d'hygiénisation (1) pour être vidé et/ou nettoyé, ledit réservoir de mélange étant apte à être en outre muni d'un élément de chauffage (39), organisé pour chauffer ledit fluide de lavage, et/ou de moyens de filtrage (68), organisés pour effectuer une opération de filtrage sur l'eau de lavage provenant de ladite station de conditionnement thermique (2).

10. Appareil d'hygiénisation (1) selon l'une quelconque des revendications 5 à 9, dans lequel lesdits moyens d'hygiénisation (6) comprennent en outre des moyens de pulvérisation (40), organisés pour pulvériser ledit fluide de lavage vers ladite station de conditionnement thermique (2), lesdits moyens de pulvérisation étant aptes à comprendre une pluralité de buses (41), chaque buse étant apte à diriger un jet pulvérisé dudit fluide de lavage dans une direction réglée vers ladite station de conditionnement thermique (2).

11. Appareil d'hygiénisation (1) selon l'une quelconque des revendications 4 à 10, et comprenant en outre des moyens de collecte organisés au-dessous de ladite paroi arrière (8) et ayant une longueur qui est telle qu'elle permet à une partie dudit fluide de lavage tombant desdits moyens d'étanchéité (21) d'être collectée et convoyée jusqu'audit réservoir de collecte et de mélange (30), et/ou des moyens d'assainissement (53) appropriés pour envoyer un fluide d'assainissement à l'intérieur de ladite station de conditionnement thermique (2), lesdits moyens d'assainissement (53) étant aptes à comprendre un cinquième réservoir (54) destiné à contenir un agent bactéricide.

12. Appareil d'hygiénisation (1) selon la revendication 11, dans lequel ledit cinquième réservoir (54) est raccordé auxdits moyens de pulvérisation (40).

13. Appareil d'hygiénisation (1) selon la revendication 11 ou 12, dans lequel lesdits moyens d'assainissement (53) comprennent un sixième réservoir (58) destiné à contenir un produit d'assainissement chimique, ledit sixième réservoir (58) étant apte à être raccordé auxdits moyens de pulvérisation (40).

14. Appareil d'hygiénisation (1) selon l'une quelconque des revendications 11 à 13, dans lequel lesdits moyens d'assainissement (53) comprennent un dispositif de production de vapeur (61) destiné à produire de la vapeur, ledit dispositif de production de vapeur étant apte à être raccordé auxdits moyens de pulvérisation (40).

15. Appareil d'hygiénisation (1) selon une quelconque revendication précédente, et comprenant en outre une unité de réfrigération (64) destinée à condenser de la vapeur d'eau présente dans un espace interne (20) de ladite station de conditionnement thermique (2) et dans ladite chambre (5), ladite unité de réfrigération (64) comprenant un élément d'évaporation (65) muni d'un enroulement (66) qui s'étend sensiblement sur toute la largeur et toute la hauteur dudit appareil d'hygiénisation (1) et qui est apte à faire en sorte qu'un écoulement de fluide de réfrigération lui étant interne vienne placer une surface dudit enroulement (66) à une température qui est inférieure à la température de l'air dans ladite chambre (5) et dans ledit espace interne (20) de ladite station de conditionnement thermique (2).

16. Appareil d'hygiénisation (1) selon une quelconque revendication précédente, et comprenant en outre des moyens de reconnaissance organisés pour que ladite station de conditionnement thermique (2) reconnaisse ledit appareil d'hygiénisation (1), lesdits moyens de reconnaissance comprenant un élément de butée (19) apte à interagir avec un micro-commutateur (19a) disposé sur ladite station de conditionnement thermique (2), ou un dispositif RFID qui est lu par une unité de lecture de ladite station de conditionnement thermique (2).

17. Appareil d'hygiénisation (1) selon la revendication 16, comprenant en outre une unité de gestion et de commande appropriée pour échanger des signaux avec une unité de gestion et de commande supplémentaire de ladite station de conditionnement thermique (2).
